# EUROPEAN PATENT SPECIFICATION

(11) **EP 4 003 220 B1**
(45) Date of publication and mention of the grant of the patent: **15.04.2026**
(21) Application number: 20746930.5
(22) Date of filing: 24.07.2020
(51) Int. Cl.: A61C 17/02, A61M 3/02

(54) **CANTILEVER-TYPE PUMP FOR AN ORAL IRRIGATOR**
FREITRAGENDE PUMPE FÜR MUNDDUSCHE
POMPE DE TYPE CANTILEVER POUR UN IRRIGATEUR ORAL

(30) Priority: 30.07.2019 US 201962880234 P
(43) Date of publication of application: 01.06.2022
(73) Proprietor: Koninklijke Philips N.V., 5656 AG Eindhoven (NL)
(72) Inventor: PHAM, Le Gia, 5656 AE Eindhoven (NL); VONDERREITH, Eric, 5656 AE Eindhoven (NL); GAO, Yan, 5656 AE Eindhoven (NL)
(74) Representative: Philips Intellectual Property & Standards
(86) International application number: PCT/EP2020/070894
(87) International publication number: WO 2021/018748

(56) References cited:
- US-A1- 2012 156 641
- US-A1- 2014 193 774
- US-A1- 2015 308 421

## Description

### Field of the Invention

The present disclosure is directed to a cantilever-type pump subassembly for an oral irrigator which provides noise and vibration reduction.

### Background

US2014/193774 discloses an irrigating device including a reservoir for storing fluid, a tip fluidly connected to the reservoir, and a pump operative to draw fluid from the reservoir and propel the fluid to the tip.

US2012/156641 discloses a vibrational frequency adjustment device comprising, as a vibrational frequency adjustment means: a first conversion means which is provided with an input-side rotating member, an output-side rotating member, and a one-way clutch for transmitting only the rotational motion in one direction of the input-side rotating member to the output-side rotating member.

US2015/308421 discloses a compressed gas motor comprising a vibration body made to vibrate by a compressed gas being guided through the compressed gas motor, and a plunger supported as in a bearing by a restoring element in spring-like manner, the vibration body and the plunger being positioned and supported such that the vibration body repeatedly hits against the plunger during the vibration and displaces it against the restoring element, whereby the motion of the plunger is utilized as the drive of the compressed gas motor.

Oral irrigators typically have a pump, which connects a fluid reservoir at one valve port and a handle with a nozzle at another valve port. The pump is driven by a motor and gear train to deliver high-pressured fluid to the nozzle tip for oral cleaning. The pump may have different operating modes selected by a user and controlled by an electronic control board of the product. The pump is often integrated to the product base or embedded in a module, which in turn is firmly secured to the product base via several attachment points along the pump body. Under different usage modes at different voltages, the pump delivers different pressures and flow rates creating pump vibration. As the pump is firmly secured to base, the transmitted vibrations create undesired noise. The vibrations may resonate further due to the product frame and/or housing, and then become more uncomfortable to some users. Accordingly, there is a continued need for oral irrigator pump assemblies which provide reduced vibration.

### Summary of the Invention

The invention is defined by the independent claims. The dependent claims define advantageous embodiments.

The present disclosure is directed to a cantilever-type pump subassembly for an oral irrigator which provides noise and vibration reduction as well as simpler assembly. The pump subassembly comprises an inlet portion arranged to receive fluid from a reservoir, an outlet portion arranged to receive a pressurized flow of the fluid, and a pressurizing portion integrally secured to the inlet portion and the outlet portion. The pump subassembly is fixedly secured only to a gearbox housing arranged within the housing of the oral irrigator by a mounting flange.

The invention is defined by the features of independent claim 1.

### Brief Description of the Drawings

In the drawings, like reference characters generally refer to the same parts throughout the different views. Also, the drawings are not necessarily to scale, emphasis instead generally being placed upon illustrating the principles of the invention.
FIG. 1 is a schematic representation of an oral irrigator according to aspects of the present disclosure.
FIG. 2 is a schematic illustration of a pump subassembly according to aspects of the present disclosure.
FIG. 3 is a schematic illustration of a cross-sectional view of a pump subassembly according to aspects of the present disclosure.
FIG. 4 is a schematic illustration of components of a pump according to aspects of the present disclosure.
FIG. 5 is a schematic illustration of the pump subassembly suspended inside the oral irrigator housing for noise and vibration suppression.
FIG. 6 is a schematic illustration of a flange of a pump subassembly according to aspects of the present disclosure.
FIG. 7A is a schematic illustration of components of a pump subassembly according to aspects of the present disclosure.
FIG. 7B is a schematic illustration of components of a pump subassembly according to aspects of the present disclosure.
FIG. 8 is a schematic illustration of a pump subassembly according to aspects of the present disclosure.
FIG. 9A is a schematic illustration of a pump subassembly according to aspects of the present disclosure.
FIG. 9B is a schematic illustration of a pump subassembly according to aspects of the present disclosure.
FIG. 10A is a schematic illustration of components of a pump subassembly according to aspects of the present disclosure.
FIG. 10B is a schematic illustration of components of a pump subassembly according to aspects of the present disclosure. FIG. 11A is a schematic illustration of components of a pump subassembly according to aspects of the present disclosure.
FIG. 11B is a schematic illustration of components of a pump subassembly according to aspects of the present disclosure.
FIG. 12 is a schematic illustration of components of a pump subassembly according to aspects of the present disclosure.

### Detailed Description

The present disclosure is directed to a cantilever-type pump subassembly for an oral irrigator which provides noise and vibration reduction as well as simpler assembly. The pump subassembly comprises an inlet portion arranged to receive fluid from a reservoir, an outlet portion arranged to receive a pressurized flow of the fluid, and a pressurizing portion integrally secured to the inlet portion and the outlet portion. The pump subassembly is fixedly secured only by a mounting flange to a gearbox housing arranged within the housing of the oral irrigator. A resilient element, such as a spring, connects the gearbox housing to the oral irrigator housing so that the gearbox is suspended within the oral irrigator housing. An elastic tie is used to connect the gearbox housing to the motor and portions of the oral irrigator housing. Additionally, the inlet cap and the outlet cap are secured to the inlet base and outlet base using locking mechanisms or fasteners which further reduce vibrations and provide for easier assembly.

Generally, to reduce undesired noise and improve a user's experience with an oral irrigator, the pump, as a component of the noise source of the oral irrigator, should generate as little as possible noise during operation while delivering sufficient pressured flow rates and fluid jet velocity within a desired range at the nozzle tip. The present disclosure is directed to a cantilever-type pump, which reduces undesired noise and vibrations by only being secured at one end to a gearbox housing, where the gearbox housing is suspended in the oral irrigator housing by a resilient element connecting the oral irrigator housing to the gearbox housing. In addition, the cantilever-type pump disclosed herein has a valve design that provides minimal operating noise and a layout of valve ports that allows connected inlet or outlet tubing to minimize vibrations and contact with surrounding product elements.

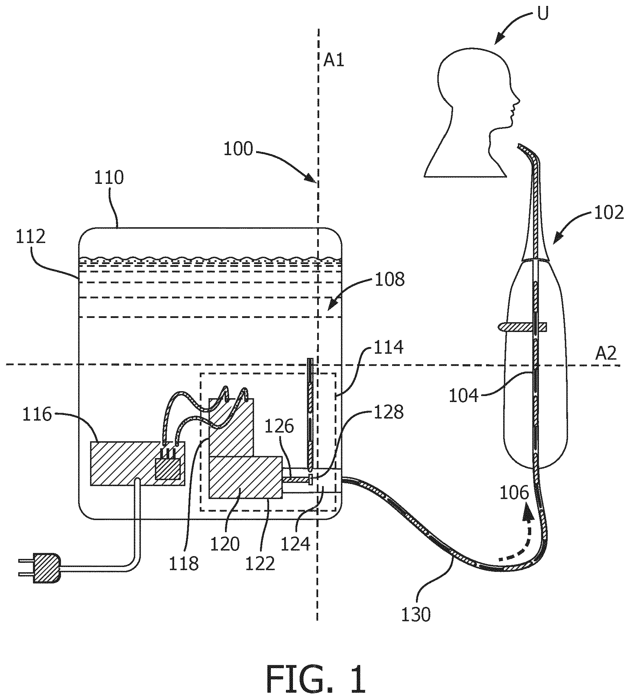

Referring to the figures, FIG. 1 is a schematic illustration of an oral irrigator system 100. Oral irrigator 100 includes irrigator tip 102 which has a handle portion and a nozzle portion. Irrigator tip 102 further includes channel 104 which extends within and through both the handle portion and the nozzle portion. Channel 104 provides flow 106 of fluid 108 to user U. Oral irrigator 100 further includes housing 110 which contains reservoir 112, pump 114, and power supply 116. Reservoir 112 contains a volume of fluid 108 which, during operation of oral irrigator 100, is directed through irrigator tip 102 into the mouth of user U. Fluid 108 can be selected from, for example, water, a water-gas mixture, oral cleansing concentrate, standard or antiseptic (alcohol based) mouthwash, or any fluid with a viscosity low enough to proceed through channel 104.

Pump 114 includes motor 118, gear box 120, gearbox housing 122, pump subassembly 124, connecting rod 126, and piston 128. Motor 118, gearbox 120, connecting rod 126, and piston 128 work in concert to create a pressurized environment that facilitates flow 106 from reservoir 112, into and through pump subassembly 124, through tether 130, into irrigator tip 102 and into user U's mouth. Tether 130 is a substantially hollow, flexible tube, having a first end and a second end. The first end of tether 130 is fixedly secured to the handle portion of the irrigator tip 102, and the second end of tether 130 is fixedly secured to pump subassembly 124. Tether 130 functions as a conduit through which flow 106 of fluid 108 precedes from reservoir 112 to irrigator tip 102 and into user U's mouth for cleaning.

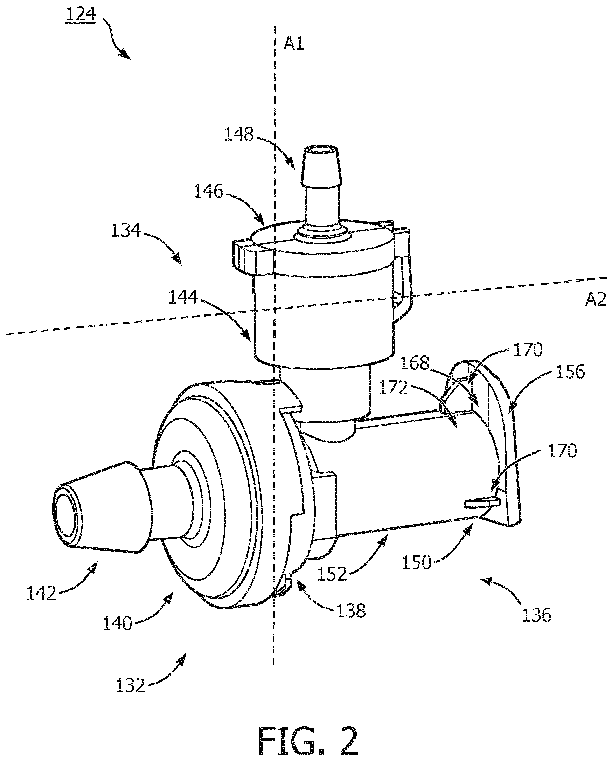

The following description should be read in light of FIGS. 2-4 which illustrate parts of an exemplary pump subassembly 124 of oral irrigator system 100. Referring to FIG. 2, the pump subassembly 124, also referred to as a pump body, comprises an inlet portion 132, an outlet portion 134, and a pressurizing portion 136, also referred to as a pump cylinder. The inlet portion 132 is arranged to receive fluid 108 from the reservoir 112 (shown in FIG. 1). The inlet portion 132 comprises an inlet base 138 which is connected to the second end 152 of the pressurizing portion 136 and the base 144 of the outlet portion 134. The inlet portion 132 also comprises an inlet cap 140 which is secured to the inlet base 138 and which has an inlet port 142 arranged to receive fluid 108 from the reservoir 112. The inlet port 142 may receive fluid 108 directly from the reservoir 112 or may connect to a hose which connects the reservoir 112 to the inlet port 142. The outlet portion 134 is arranged to receive a pressurized flow 106 of the fluid 108 generated by the pump 114. The outlet portion 134 has an outlet base 144 integrally connected to the pressurized portion 136 and the inlet portion 132, and the outlet portion 134 has an outlet cap 146 which is secured to the outlet base 144. The outlet cap 146 comprises an outlet port 148 which is arranged to receive the pressurized flow 106 of fluid 108. The outlet port 148 may directly connect to the tether 130 or may be connected to the tether 130 by an additional connector or hose, such as a coiled tether, and the outlet port 148 sends the pressurized flow 106 of fluid 108 to the tether 130. Channel 104 (shown in FIG. 1) is in fluid communication with the inlet portion 132, the outlet portion 134, and the pressurizing portion 136 of pump subassembly 124.

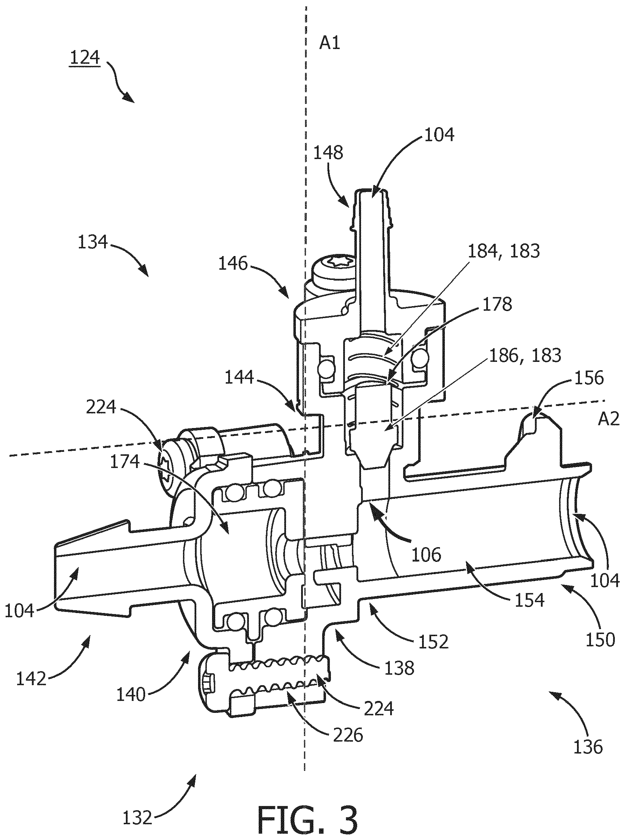

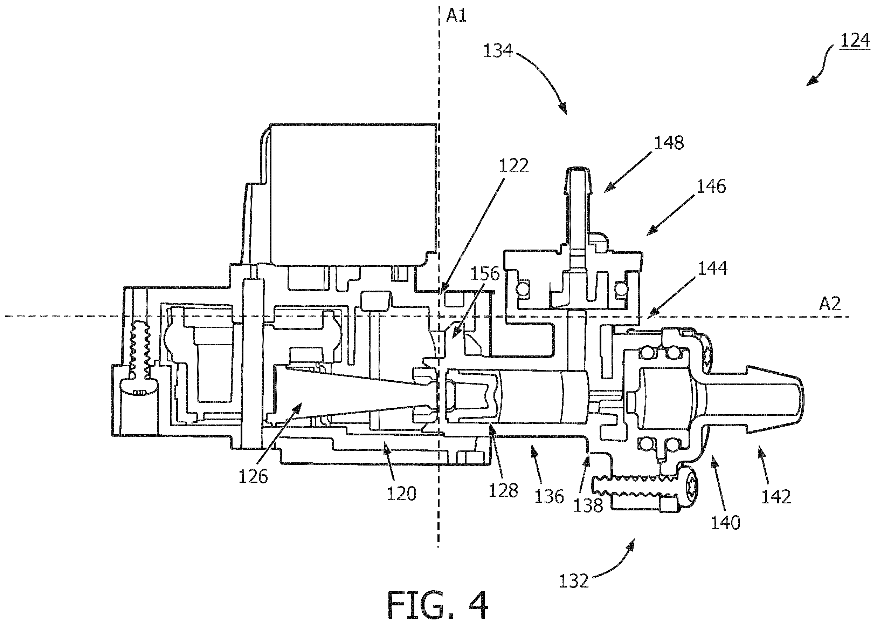

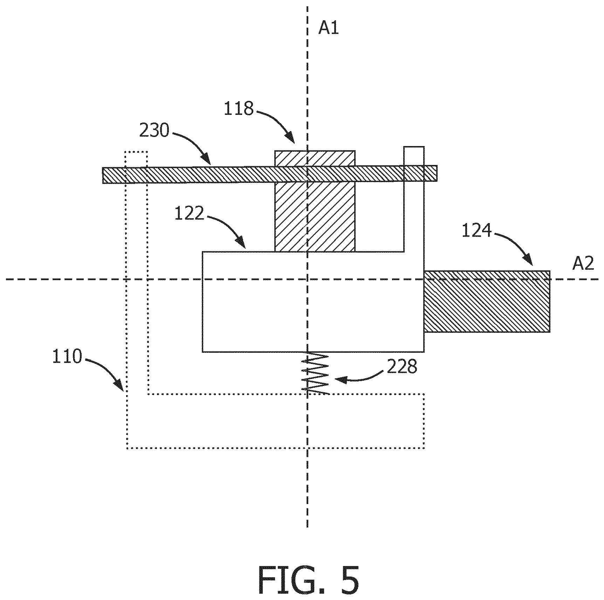

FIG. 3 is a cross sectional view of the components of an exemplary pump subassembly 124. The pressurized portion 136 has a first end 150, a second end 152, and a through bore 154 arranged between the first end 150 and the second end 152. As shown in FIGS. 3 and 4, the through bore 154 is arranged to receive a piston 128 and a connecting rod 126 of the pump 114 which creates a pressurized flow 106. As shown in FIG. 2, the first end 150 of the pressurized portion 136 has a mounting flange 156. The pump subassembly 124 is fixedly secured to the gearbox housing 122 of gearbox 120 using the mounting flange 156. The pump subassembly 124 is only secured to other components of the pump 114 at the pressurized portion 136 where the pump subassembly 124 is secured to the gearbox housing 122 (shown in FIG. 4). The inlet port 142 and the outlet port 148 of the pump subassembly 124 also make contact with the reservoir 112 and tether 130, respectively, or hoses which connect to the reservoir 12 and tether 130 (shown in FIG. 1). As shown in FIG. 5, the gear box housing 122, which is secured to the pump subassembly 124, is suspended in the irrigator housing 110 via a resilient element 228, such as a spring. The resilient element 228 connects the irrigator housing 110 to the gearbox housing 122, and dampens vibrations from the gearbox 120 and pump subassembly 124, particularly vibrations along the A1 axis. Additionally, the gearbox housing 122 is secured using an elastic tie 230 to the motor 118 and the irrigator housing 110. As an example, the elastic tie 230 may be a three prong rubber tie which has multiple connectors which connect the gearbox housing 122 to the motor 118 and the oral irrigator housing 110. The elastic tie 230 may be made of a flexible material which can deform in three dimensions to further dampen noise and vibrations from the motor 188, gearbox 120, and pump subassembly 124.

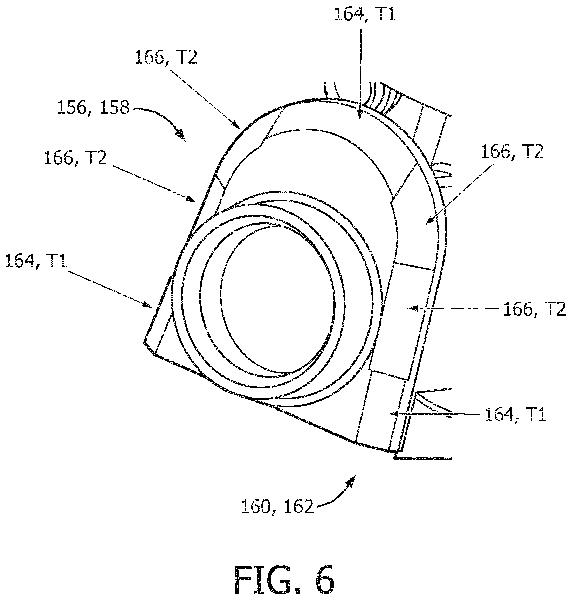

As shown in FIG. 6, the mounting flange 156, which connects the pump subassembly 124 to the gearbox housing 122, has a first face 158 which has an outer chamfer 160 which contains a plurality of chamfered surfaces 162. The plurality of chamfered surfaces 162 includes a plurality of contact surfaces 164 and a plurality of proximal surfaces 166. In an example, the outer chamfer 160 contains three contact surfaces 164. The plurality of contact surfaces 164 have a first thickness T1 which is greater than the thickness of the plurality proximal surfaces 166, a second thickness T2. The contact surfaces 164 ensure a tight fit between the pressurizing portion 136 of the pump subassembly 124 and the gearbox housing 122, which reduces vibrations and noise in the oral irrigator system. The second face 168 (shown in FIG. 2) of the mounting flange 156 has a plurality of reinforced ribs 170. The reinforced ribs 170 are secured between the second face 168 of the mounting flange 156 and an outer circumferential surface 172 of the pressurized portion 136. The gearbox housing 122 (shown in FIG. 4) may also include features for noise and vibration reduction, for example, including nominal wall thickness at approximately 2 mm, and extensive rib and stiffening features to reduce vibration in the structure.

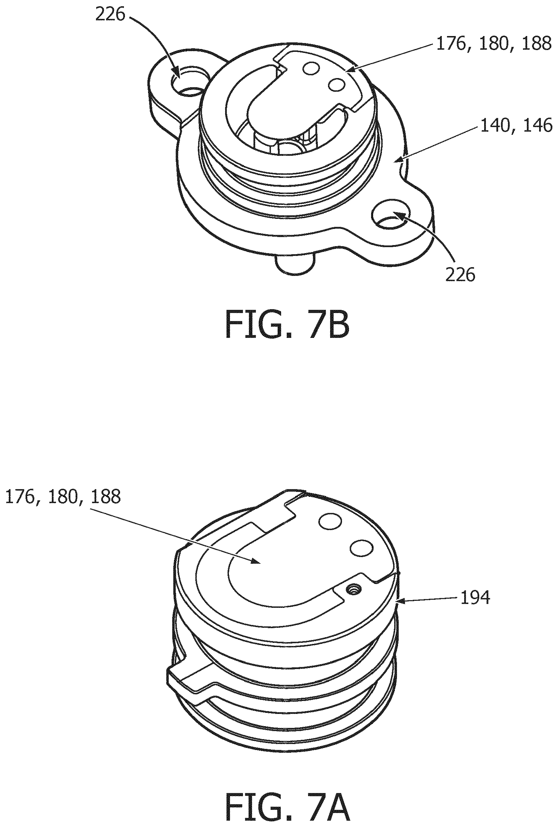

Referring to FIG. 3, the inlet portion 132 further comprises an inlet cavity 174 arranged to receive an inlet valve 176 (shown in FIG. 7A and FIG. 7B), and the outlet portion 134 further comprises an outlet cavity 178 (shown in FIG. 3) arranged to receive an outlet valve 180 (shown in FIG. 7A and FIG. 7B). The inlet valve 176 and the outlet valve 180 may be, for example, reed check valves, film valves, or leaf valves. The inlet valves open under suction pressure, and the outlet valves open under compression pressure. The valves close in other phases of the operation of pump 114. In an example, the film valves are die cut thin films 182 (not shown in Figures), which are low cost and quiet due to their very low mass and due to their flexibility in the small opening of the valve. In another example, the outlet valve 180 or the inlet valve 176 is a spring valve 183 comprising a resilient element 184, for example, a spring, and an elastic head element 186, for example, a rubber valve head (shown in FIG. 3). When the flow 106 provides enough pressure to overcome the spring force, the elastic head element 186 is moved to open the valve. When there is not enough pressure, the elastic head element 186 seals the flow by making contact with an inner surface of the channel 104. The spring element 183 has an elastic relationship between the elastic head element 186 and fluid flow 106. An advantage of using a spring valve 183 is that it creates a strong seal, and an advantage of the reed check valves, film valves, or leaf valves is that they are quiet. In another example, the outlet valve 180 or the inlet valve 176 (shown in FIG. 7A and FIG. 7B) is a reed check valve 188, which is secured, for example, by heat staking, to a core support element 194, the outlet cap 146, or the inlet cap 140 to avoid misalignment or dislodgement during assembly.

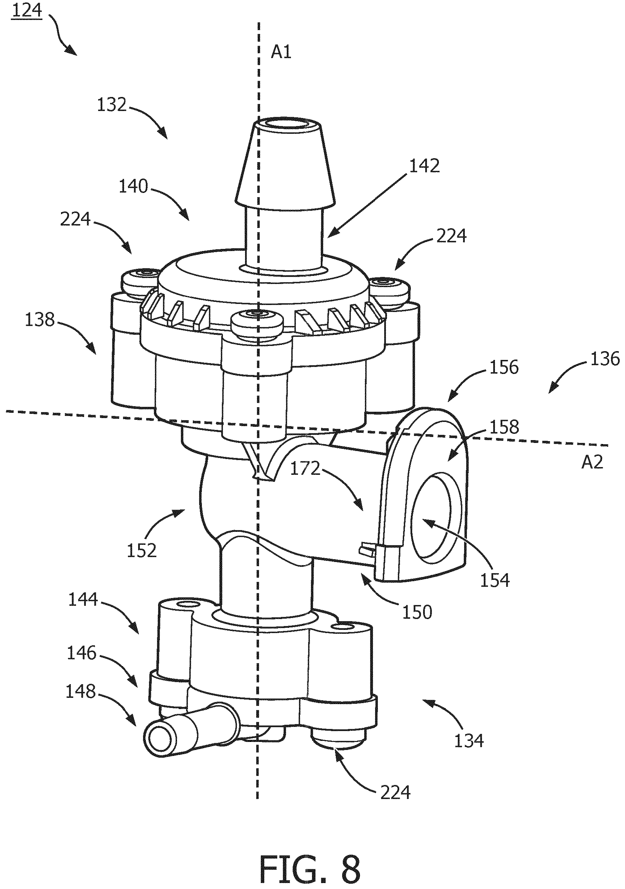

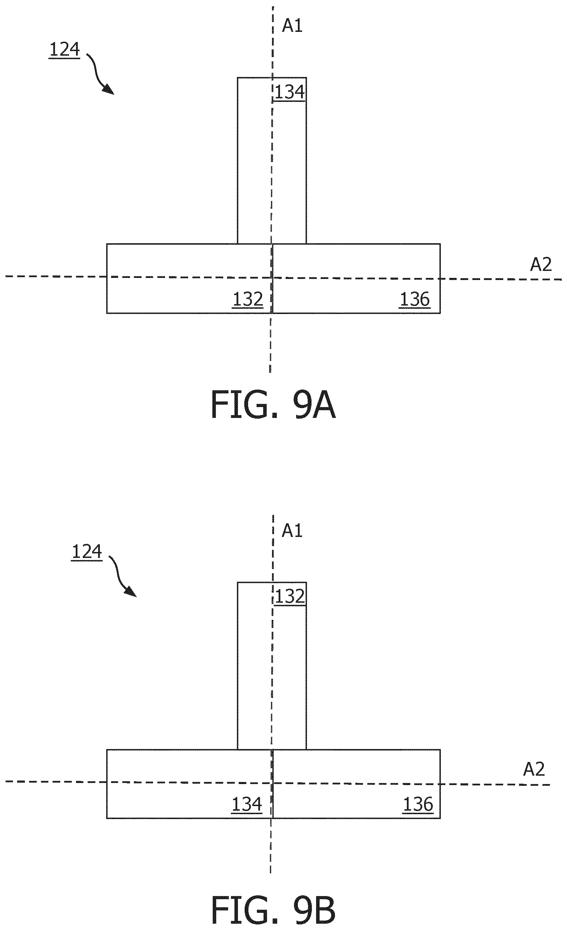

An exemplary layout of the components of the pump subassembly is shown in FIG. 8. The components of the pump subassembly 124, the inlet portion 132, the outlet portion 134, and the pressurizing portion 136, may be arranged so that the inlet portion 132 and the outlet portion 134 are arranged about a first axis A1. In this example, the pressurizing portion 136 is arranged about a second axis A2 such that the through bore 154 is arranged about and substantially parallel with the second axis A2. The first axis A1 and the second axis A2 are imaginary and orthogonal to each other. It should be appreciated that the pump subassembly 124 shown in FIG. 8 could also be arranged horizontally, so that inlet portion 132 and the outlet portion 134 are arranged about the A2 axis, and the pressurizing portion 136 is arranged about the A1 axis. FIG. 9A provides a schematic illustration of the pump subassembly 124 shown in FIG. 3. The inlet portion 132 is arranged about a first axis A1, and the pressurizing portion 136 is arranged about the first axis A1 such that through bore 154 is arranged about and substantially parallel with the first axis A1. The outlet portion 134 is arranged about a second axis A2. The first axis A1 and the second axis A2 are imaginary and orthogonal to each other. This orientation allows connecting tubing from the reservoir 112 to the pump subassembly 124 and from the outlet port 148 to the tether 130 and/or irrigator tip 102 to have enough space for vibrations without resonance and without striking the surrounding housing 110 (which increases noise). In another example, shown in FIG. 9B, the outlet portion 134 is arranged about a first axis A1, and the pressurizing portion 136 is arranged about a first axis A1 such that through bore 154 is arranged about and substantially parallel with the first axis A1. In this example, the inlet portion 132 is arranged about a second axis A2. The first axis A1 and the second axis A2 are imaginary and orthogonal to each other. This layout reduces vibration from the pump 114 to the housing 110 if there is enough space vertically for connecting tubing from the outlet port 148 to the tether 130 and/or irrigator tip 102 to have a soft connection with the pump 114. It should be appreciated that although reservoir 112 is shown in FIG. 1 as located above the pump 114 along the A1 axis, the reservoir 112 may also be located adjacent to the pump 114 along the A2 axis.

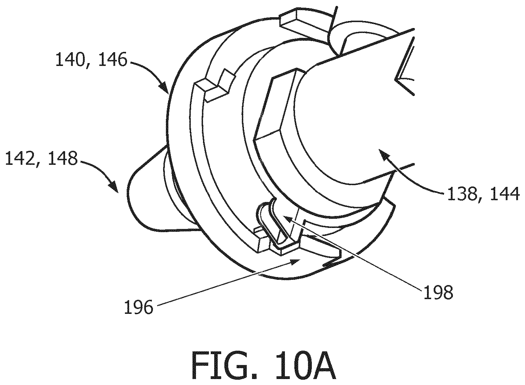

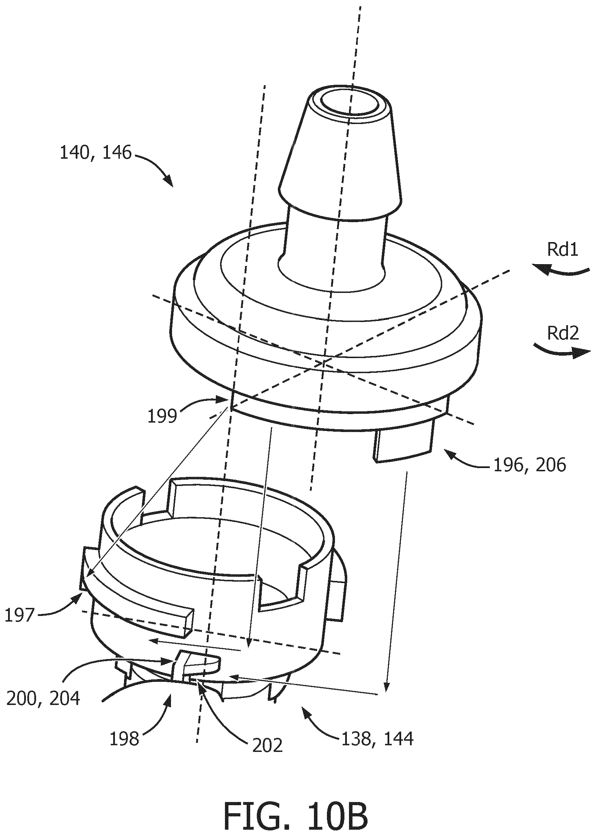

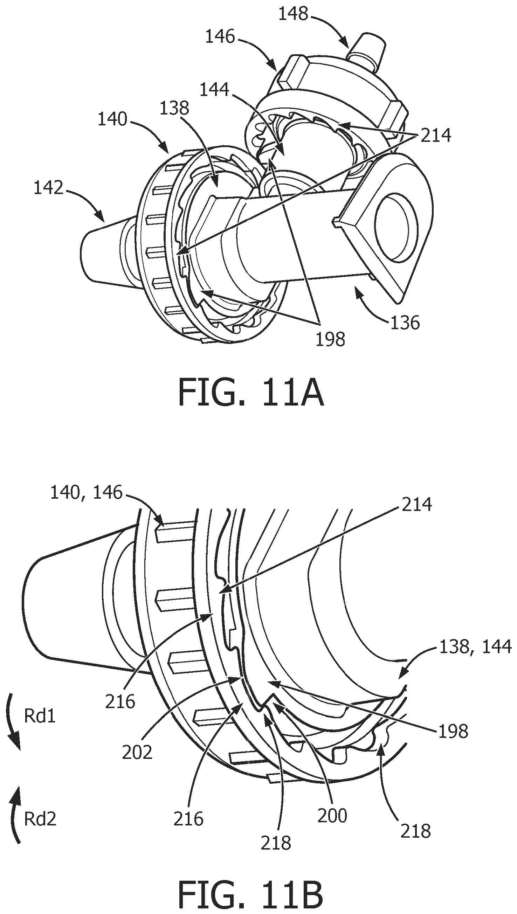

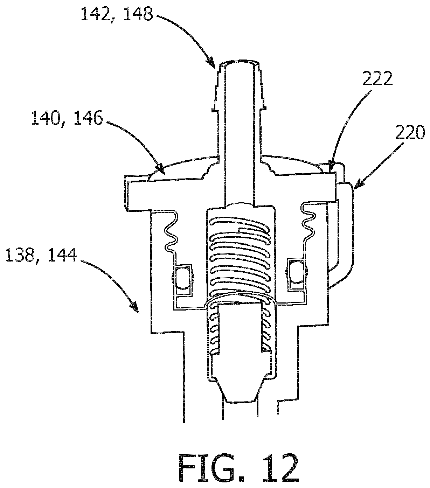

FIGS. 10A, 10B, 11A, 11B, and 12 illustrate exemplary ways that the inlet cap 140 and outlet cap 146 may be secured to the inlet base 138 and outlet base 144, respectively. As shown in FIGS. 10A and 10B, the inlet cap 140 or outlet cap 146 may be secured to the inlet base 138 or outlet base 144, respectively, by the connection between a lock tab 196 integrally connected to the inlet cap 140 or outlet cap 146 and a locking feature 198 integrally connected to the inlet base 138 or outlet base 144. As shown in FIG. 10B, the locking feature 198 has a locking surface 200 and an engagement surface 202. The engagement surface 202 is chamfered and allows the locking feature 198 to slide past the lock tab 196 as the inlet/outlet cap 140/146 is rotated in a first rotational direction RD1. The lock tab 196 moves over the chamfered engagement surface 202 and is deformed as the engagement surface 202 moves across the lock tab 196 in a second rotational direction RD2. The locking surface 200 has a first face 204 which faces the first rotational direction RD1. When the inlet/out cap 140/146 is secured to the inlet/out base 138/144, the first face 206 of the lock tab 196, which faces in the RD2 direction, makes contact with the first face 204 of the locking surface 200 and blocks the cap from rotating in the RD2 direction. The inlet/outlet cap 140/146 may also be blocked from rotating in the first rotational direction RD1 by a protruding surface 197 on the inlet/outlet base 138/144 which engages with and blocks an indented surface 199 on the inlet/outlet cap 140/146. It should be appreciated that the inlet/outlet base 138/144 may have more than one locking feature 198 and/or protruding surface and the inlet/outlet cap 140/146 may have more than one lock tab 196 and/or indented surface. FIGS. 11A and 11B illustrate another exemplary mechanism to secure the inlet cap 140 to the inlet base 138 and/or the outlet cap 146 to the outlet base 144. The inlet/outlet base 138/144 is connected to a locking feature 198, which has a curved engagement surface 202 and a locking surface 200 (shown in FIG. 11B). The locking feature 198 engages with lock teeth 214 on the inlet/outlet cap 140/146 so that the inlet/outlet cap 140/146 is free to rotate in a first direction RD1 but not a second direction RD2 when the locking feature 198 and lock teeth 214 are engaged with each other. The locking feature 198 and lock teeth 214 operate as a ratchet mechanism so that the engagement surfaces 202 slide past one or more sliding faces 216 of the lock teeth 214 when the cap 140/146 is rotated in the first rotational direction RD1. When the inlet/outlet cap 140/146 is rotated in a second rotational direction RD2, the locking surface 200 of the locking feature 198 contacts and is blocked by blocking faces 218 of the lock teeth 214. FIG. 12 is a schematic illustration of an inlet cap 140 or outlet cap 146 which is secured to the inlet base 138 or outlet base 144, respectively, using a deformable engagement feature 220. The deformable engagement feature 220 contacts a tab 222 connected to the inlet/outlet cap 140/146 when the inlet/outlet cap 140/146 is secured to the inlet/outlet base 138/144.

The inlet/outlet cap 140/146 and inlet/outlet base 138/144 may also be secured by other methods such as screw fasteners, glue, or ultra-sonic welding. For example, FIG. 3, FIG. 7B, and FIG. 8 provide illustrations of screw fasteners 224 and through bores 226 for screw fasteners 224.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only. The invention is defined by the features of independent claim 1.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. As used herein in the specification and in the claims, "or" should be understood to have the same meaning as "and/or" as defined above.

## Claims

1. An oral irrigator (100) comprising a pump subassembly (124) arranged within a housing (110) of the oral irrigator (100), the pump subassembly (124) comprising:
an inlet portion (132) arranged to receive fluid (108) from a reservoir (112);
an outlet portion (134) integrally secured to the inlet portion, the outlet portion arranged to receive a pressurized flow (106) of the fluid; and,
a pressurizing portion (136) integrally secured to the inlet portion and the outlet portion, the pressurizing portion comprising a first end (150) having a mounting flange (156),
wherein the pump subassembly (124) is fixedly secured only to a gearbox housing (122) arranged within the housing (110) of the oral irrigator (100) by the mounting flange (156); and,
**characterized in that** the mounting flange comprises a first face (158) having an outer chamfer (160), the outer chamfer having a plurality of chamfered surfaces (162), the plurality of chamfered surfaces including a plurality of contact surfaces (164) and a plurality of proximal surfaces (166), wherein the plurality of contact surfaces have a first thickness (T1) and the plurality of proximal surfaces have a second thickness (T2) where the first thickness is greater than the second thickness.

2. The oral irrigator (100) of claim 1, wherein the gearbox housing (122) is connected to the housing (110) of the oral irrigator (100) by a resilient element (228), and wherein the gearbox housing (122) is connected to a motor (118) and the housing of the oral irrigator by an elastic tie (230).

3. The oral irrigator (100) of claim 1, wherein the inlet portion comprises:
an inlet base (138) integrally connected to the pressurizing portion and the outlet portion; and,
an inlet cap (140) secured to the inlet base, the inlet cap comprising an inlet port (142) arranged to receive the fluid; and wherein the outlet portion comprises:
an outlet base (144) integrally connected to the pressurizing portion and the inlet portion; and,
an outlet cap (146) secured to the inlet base, the outlet cap comprising an outlet port (148) arranged to receive the pressurized flow of the fluid.

4. The oral irrigator (100) of claim 1, wherein the pressurizing portion further comprises an outer circumferential surface (172), and the mounting flange further comprises a second face (168) and a plurality of reinforced ribs (170) secured between the second face of the mounting flange and the outer circumferential surface of the pressurizing portion.

5. The oral irrigator (100) of claim 1, wherein the inlet portion further comprises an inlet cavity (174) arranged to receive an inlet valve (176), and wherein the outlet portion further comprises an outlet cavity (178) arranged to receive an outlet valve (180).

6. The oral irrigator (100) of claim 5, wherein the inlet valve (176) and the outlet valve (180) are selected from: a reed check valve, a film valve, and a leaf valve.

7. The oral irrigator (100) of claim 5, wherein the outlet valve (180) is a spring valve (183) comprising a resilient element (184) and an elastic head element (186).

8. The oral irrigator (100) of claim 1, wherein the inlet portion (132) and the pressurizing portion (136) are arranged about a first axis (A1), and the outlet portion (134) is arranged about a second axis (A2), wherein the first axis is orthogonal to the second axis.

9. The oral irrigator (100) of claim 1, wherein the outlet portion (134) and the pressurizing portion (136) are arranged about a first axis (A1), and the inlet portion (132) is arranged about a second axis (A2), wherein the first axis is orthogonal to the second axis.

10. The oral irrigator (100) of claim 1, wherein the pressurizing portion (136) is arranged about a first axis (A1), and the inlet portion (132) and the outlet portion (134) are arranged about a second axis (A2), wherein the first axis is orthogonal to the second axis.

11. The oral irrigator (100) of claim 3, wherein the inlet base (138) or the outlet base (144) further comprises a locking feature (198) comprising an engagement surface (202) and a locking surface (200) arranged to engage the inlet cap (140) or outlet cap (146) such that the inlet cap or the outlet cap is arranged to rotate in a first direction (RD1) but not a second direction (RD2) when the locking surface and the engagement surface are in contact with a lock tab (64) or lock teeth (214) on the inlet cap or outlet cap.

12. The oral irrigator (100) of claim 3, wherein the inlet base (138) or outlet base (144) further comprises a deformable engagement feature (220) arranged to secure the inlet cap (140) or outlet cap (146) to the inlet base or outlet base.

13. The oral irrigator (100) of claim 3, wherein the outlet cap (146) is secured to the outlet base using ultrasonic welding or glue; or wherein the inlet cap (140) is secured to the inlet base using, screw fasteners, ultrasonic welding, or glue.

14. The oral irrigator (100) of claim 6, wherein the reed check valve (176, 180, 188) is fixedly secured to a core support element (194) of the reed check valve, an outlet cap (146), or an inlet cap (140).

## Patentansprüche

1. Munddusche (100) mit einer Pumpenbaugruppe (124), die in einem Gehäuse (110) der Munddusche (100) angeordnet ist, wobei die Pumpenbaugruppe (124) Folgendes umfasst:
einen Einlassabschnitt (132), der so angeordnet ist, dass er Fluid (108) aus einem Behälter (112) aufnimmt;
einen Auslassabschnitt (134), der fest an dem Einlassabschnitt fixiert ist, wobei der Auslassabschnitt so angeordnet ist, dass er einen unter Druck stehenden Strom (106) des Fluids aufnimmt; und
einen Druckbeaufschlagungsabschnitt (136), der fest an dem Einlassabschnitt und dem Auslassabschnitt fixiert ist, wobei der Druckbeaufschlagungsabschnitt ein erstes Ende (150) umfasst, das einen Montageflansch (156) aufweist,
wobei die Pumpenbaugruppe (124) mittels des Montageflansches (156) nur fest mit einem im Gehäuse (110) der Munddusche (100) angeordneten Getriebegehäuse (122) verbunden ist; und
**dadurch gekennzeichnet, dass** der Montageflansch eine erste Fläche (158) umfasst, die eine äußere Fase (160) aufweist, die äußere Fase mehrere gefaste Oberflächen (162) aufweist, die mehreren gefasten Oberflächen mehrere proximale Kontaktoberflächen (164) und mehrere proximale Oberflächen (166) umfassen, wobei die mehreren Kontaktoberflächen eine erste Dicke (T1) aufweisen und die mehreren proximalen Oberflächen eine zweite Dicke (T2) aufweisen, wobei die erste Dicke größer als die zweite Dicke ist.

2. Munddusche (100) nach Anspruch 1, wobei das Getriebegehäuse (122) mittels eines elastischen Elements (228) mit dem Gehäuse (110) der Munddusche (100) verbunden ist und wobei das Getriebegehäuse (122) mittels eines elastischen Bands (230) mit einem Motor (118) und dem Gehäuse der Munddusche verbunden ist.

3. Munddusche (100) nach Anspruch 1, wobei der Einlassabschnitt Folgendes umfasst:
einen Einlasssockel (138), der fest mit dem Druckbeaufschlagungsabschnitt und dem Auslassabschnitt verbunden ist; und
eine am Einlasssockel fixierte Einlasskappe (140), wobei die Einlasskappe eine Einlassöffnung (142) umfasst, die dazu angeordnet ist, das Fluid aufzunehmen; und wobei der Auslassabschnitt Folgendes umfasst:
einen Auslasssockel (144), der fest mit dem Druckbeaufschlagungsabschnitt und dem Einlassabschnitt verbunden ist; und
eine an der Einlassbasis fixierte Auslasskappe (146), wobei die Auslasskappe eine Auslassöffnung (148) aufweist, die dazu angeordnet ist, den unter Druck stehenden Strom des Fluids aufzunehmen.

4. Munddusche (100) nach Anspruch 1, wobei der Druckbeaufschlagungsabschnitt ferner eine äußere Umfangsoberfläche (172) umfasst und der Montageflansch ferner eine zweite Fläche (168) und mehrere verstärkte Rippen (170) aufweist, die zwischen der zweiten Fläche des Montageflansches und der Außenumfangsoberfläche des Druckbeaufschlagungsabschnitts fixiert sind.

5. Munddusche (100) nach Anspruch 1, wobei der Einlassabschnitt ferner einen Einlassraum (174) umfasst, der dazu angeordnet ist, ein Einlassventil (176) aufzunehmen, und wobei der Auslassabschnitt ferner einen Auslassraum (178) umfasst, der dazu angeordnet ist, ein Auslassventil (180) aufzunehmen.

6. Munddusche (100) nach Anspruch 5, wobei das Einlassventil (176) und das Auslassventil (180) ausgewählt sind aus: einem Membranrückschlagventil, einem Folienventil und einem Blattventil.

7. Munddusche (100) nach Anspruch 5, wobei das Auslassventil (180) ein Federventil (183) ist, das ein elastisches Element (184) und ein elastisches Kopfelement (186) umfasst.

8. Munddusche (100) nach Anspruch 1, wobei der Einlassabschnitt (132) und der Druckbeaufschlagungsabschnitt (136) um eine erste Achse (A1) angeordnet sind und der Auslassabschnitt (134) um eine zweite Achse (A2) angeordnet ist, wobei die erste Achse orthogonal zur zweiten Achse ist.

9. Munddusche (100) nach Anspruch 1, wobei der Auslassabschnitt (134) und der Druckbeaufschlagungsabschnitt (136) um eine erste Achse (A1) angeordnet sind und der Einlassabschnitt (132) um eine zweite Achse (A2) angeordnet ist, wobei die erste Achse orthogonal zur zweiten Achse ist.

10. Munddusche (100) nach Anspruch 1, wobei der Druckbeaufschlagungsabschnitt (136) um eine erste Achse (A1) angeordnet ist und der Einlassabschnitt (132) und der Auslassabschnitt (134) um eine zweite Achse (A2) angeordnet sind, wobei die erste Achse orthogonal zur zweiten Achse ist.

11. Munddusche (100) nach Anspruch 3, wobei die Einlassbasis (138) oder die Auslassbasis (144) ferner ein Verriegelungselement (198) aufweist, das eine Eingriffsoberfläche (202) und eine Verriegelungsoberfläche (200) umfasst, die so angeordnet sind, dass sie mit der Einlasskappe (140) oder Auslasskappe (146) in Eingriff stehen, sodass die Einlasskappe oder die Auslasskappe angeordnet ist, in einer ersten Richtung (RD1), aber nicht in einer zweiten Richtung (RD2) zu rotieren, wenn die Verriegelungsoberfläche und die Eingriffsoberfläche mit einer Verriegelungsnase (64) oder Verriegelungszähnen (214) an der Einlasskappe oder Auslasskappe in Kontakt stehen.

12. Munddusche (100) nach Anspruch 3, wobei die Einlassbasis (138) oder Auslassbasis (144) ferner ein verformbares Eingriffselement (220) umfasst, das dazu angeordnet ist, die Einlasskappe (140) oder Auslasskappe (146) an der Einlassbasis oder Auslassbasis zu fixieren.

13. Munddusche (100) nach Anspruch 3, wobei die Auslasskappe (146) unter Verwendung von Ultraschallschweißen oder Klebstoff an der Auslassbasis fixiert ist; oder wobei die Einlasskappe (140) unter Verwendung von Schraubbefestigungen, Ultraschallschweißen oder Klebstoff an der Einlassbasis fixiert ist.

14. Munddusche (100) nach Anspruch 6, wobei das Membranrückschlagventil (176, 180, 188) starr an einem Kernstützelement (194) des Membranrückschlagventils, einer Auslasskappe (146) oder einer Einlasskappe (140) fixiert ist.

## Revendications

1. Irrigateur buccal (100) comprenant un sous-ensemble de pompe (124) agencé à l'intérieur d'un boîtier (110) de l'irrigateur buccal (100), le sous-ensemble de pompe (124) comprenant :
une partie d'entrée (132) agencée pour recevoir le fluide (108) d'un réservoir (112) ;
une partie de sortie (134) fixée d'un seul tenant à la partie d'entrée, la partie de sortie étant agencée pour recevoir un flux sous pression (106) du fluide ; et,
une partie de pressurisation (136) fixée d'un seul tenant à la partie d'entrée et à la partie de sortie, la partie de pressurisation comprenant une première extrémité (150) présentant une bride de montage (156),
dans lequel le sous-ensemble de pompe (124) est fixé solidement seulement à un boîtier de transmission (122) agencé à l'intérieur du boîtier (110) de l'irrigateur buccal (100) par la bride de montage (156) ; et,
**caractérisé en ce que** la bride de montage comprend une première face (158) présentant un chanfrein extérieur (160), le chanfrein extérieur présentant une pluralité de surfaces chanfreinées (162), la pluralité de surfaces chanfreinées incluant une pluralité de surfaces de contact (164) et une pluralité de surfaces proximales (166), dans lesquelles la pluralité de surfaces de contact présente une première épaisseur (T1) et la pluralité de surfaces proximales présente une seconde épaisseur (T2) où la première épaisseur est supérieure à la seconde épaisseur.

2. Irrigateur buccal (100) selon la revendication 1, dans lequel le boîtier de transmission (122) est relié au boîtier (110) de l'irrigateur buccal (100) par un élément élastique (228), et dans lequel le boîtier de transmission (122) est relié à un moteur (118) et au boîtier de l'irrigateur buccal par une attache élastique (230).

3. Irrigateur buccal (100) selon la revendication 1, dans lequel la partie d'entrée comprend :
une base d'entrée (138) reliée d'un seul tenant à la partie de pressurisation et à la partie de sortie ; et,
un bouchon d'entrée (140) fixé à la base d'entrée, le bouchon d'entrée comprenant un orifice d'entrée (142) agencé pour recevoir le fluide ; et dans lequel la partie de sortie comprend :
une base de sortie (144) reliée d'un seul tenant à la partie de pressurisation et à la partie d'entrée ; et,
un bouchon de sortie (146) fixé à la base d'entrée, le bouchon de sortie comprenant un orifice de sortie (148) agencé pour recevoir le flux sous pression du fluide.

4. Irrigateur buccal (100) selon la revendication 1, dans lequel la partie de pressurisation comprend en outre une surface circonférentielle extérieure (172), et la bride de montage comprend en outre une seconde face (168) et une pluralité de nervures renforcées (170) fixées entre la seconde face de la bride de montage et la surface circonférentielle extérieure de la partie de pressurisation.

5. Irrigateur buccal (100) selon la revendication 1, dans lequel la partie d'entrée comprend en outre une cavité d'entrée (174) agencée pour recevoir une vanne d'entrée (176), et dans lequel la partie de sortie comprend en outre une cavité de sortie (178) agencée pour recevoir une vanne de sortie (180).

6. Irrigateur buccal (100) selon la revendication 5, dans lequel la vanne d'entrée (176) et la vanne de sortie (180) sont choisies parmi : un clapet anti-retour à clapet, une vanne à film et une vanne à feuille.

7. Irrigateur buccal (100) selon la revendication 5, dans lequel la vanne de sortie (180) est une vanne à ressort (183) comprenant un élément élastique (184) et un élément de tête élastique (186).

8. Irrigateur buccal (100) selon la revendication 1, dans lequel la partie d'entrée (132) et la partie de pressurisation (136) sont agencées autour d'un premier axe (A1), et la partie de sortie (134) est agencée autour d'un second axe (A2), dans lequel le premier axe est orthogonal au second axe.

9. Irrigateur buccal (100) selon la revendication 1, dans lequel la partie de sortie (134) et la partie de pressurisation (136) sont agencées autour d'un premier axe (A1), et la partie d'entrée (132) est agencée autour d'un second axe (A2), dans lequel le premier axe est orthogonal au second axe.

10. Irrigateur buccal (100) selon la revendication 1, dans lequel la partie de pressurisation (136) est agencée autour d'un premier axe (A1), et la partie d'entrée (132) et la partie de sortie (134) sont agencées autour d'un second axe (A2), dans lequel le premier axe est orthogonal au second axe.

11. Irrigateur buccal (100) selon la revendication 3, dans lequel la base d'entrée (138) ou la base de sortie (144) comprend en outre un élément de verrouillage (198) comprenant une surface de mise en prise (202) et une surface de verrouillage (200) agencées pour venir en prise avec le bouchon d'entrée (140) ou le bouchon de sortie (146) de sorte que le bouchon d'entrée ou le bouchon de sortie soit agencé de manière à tourner dans une première direction (RD1), mais pas dans une seconde direction (RD2) lorsque la surface de verrouillage et la surface de mise en prise sont en contact avec une languette de verrouillage (64) ou des dents de verrouillage (214) sur le bouchon d'entrée ou le bouchon de sortie.

12. Irrigateur buccal (100) selon la revendication 3, dans lequel la base d'entrée (138) ou la base de sortie (144) comprend en outre un élément de mise en prise déformable (220) agencé pour fixer le bouchon d'entrée (140) ou le bouchon de sortie (146) à la base d'entrée ou à la base de sortie.

13. Irrigateur buccal (100) selon la revendication 3, dans lequel le bouchon de sortie (146) est fixé à la base de sortie à l'aide d'un soudage par ultrasons ou de colle ; ou dans lequel le bouchon d'entrée (140) est fixé à la base d'entrée à l'aide de fixations à vis, d'un soudage par ultrasons ou de colle.

14. Irrigateur buccal (100) selon la revendication 6, dans lequel le clapet anti-retour à clapet (176, 180, 188) est fixé solidement à un élément de support central (194) du clapet anti-retour à clapet, à un bouchon de sortie (146) ou à un bouchon d'entrée (140).
